(19) Europäisches Patentamt
European Patent Office
Office européen des brevets

(11) Publication number: **0 184 120 A2**

# (12) EUROPEAN PATENT APPLICATION

(21) Application number: 85115020.1

(22) Date of filing: 28.11.85

(51) Int. Cl.⁴: C 07 D 405/14
C 07 D 405/12, G 01 N 33/533

(30) Priority: 29.11.84 US 676123

(43) Date of publication of application: 11.06.86 Bulletin 86/24

(84) Designated Contracting States: BE CH DE FR GB IT LI NL SE

(71) Applicant: ABBOTT LABORATORIES
14th Street and Sheridan Road North St
North Chicago Illinois 60064(US)

(72) Inventor: Walters, Roland L.
724 Concord Lane
Barrington, IL. 60010(US)

(72) Inventor: Dodge, Robert H.
15683 W. Idlewood Lane
Libertyville, IL. 60048(US)

(72) Inventor: Kaplan, Michael J.
2650 Brookwood Way Apt. 317
Rolling Meadows, IL. 60008(US)

(74) Representative: Josif, Albert et al,
MODIANO, JOSIF, PISANTY & STAUB Via Meravigli, 16
I-20123 Milan(IT)

## (54) Tracers for disopyramide assay and immunogens to raise antibody.

(57) This invention relates to an improved fluorescent polarization assay for disopyramide, and to improved reagents (tracer compounds and immunogen) for use in the assay. The 10 tracer compounds are the reaction product of p- or m-aminodisopyramide with 10 fluorescein compounds. The tracers form complexes with antiserum specific to disopyramide, in proportion to the relative concentration of the tracer with respect to the disopyramide present in the sample. Excellent results are obtained when such a tracer compound is used in fluorescent polarization assay for disopyramide, with greater speed and less cross-reactivity than prior art methods of immunoassay.

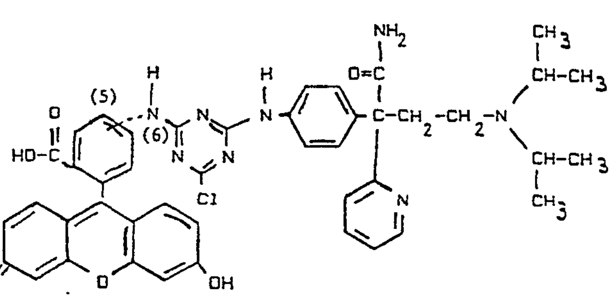

N-4-CHLORO-6-{p-[1-CARBAMYL-3-(DIISOPROPYLAMINO)-1-(2-PYRIDYL)PROPYL]ANILINO}TRIAZIN-2-YL-x-AMINOFLUORESCEIN.

(x=5, TRACER NO. 1; x=6, TRACER NO. 2)

FIGURE 9.

EP 0 184 120 A2

Croydon Printing Company Ltd

# TRACERS FOR DISOPYRAMIDE ASSAY AND IMMUNOGENS TO RAISE ANTIBODY

## BACKGROUND OF THE INVENTION

### 1. Technical Field

This invention relates to diagnostic assays, and to immunogens to raise antibody. More specifically, it relates to (1) reagents (e.g., tracer compounds) for determining the amount of disopyramide in fluids, especially biological fluids such as serum, saliva, urine, plasma, spinal fluid or amniotic fluid; (2) immunogens to raise antibody for use with the reagents; (3) synthesis methods (methods of making the reagents and immunogen); and (4) analytical methods for carrying out disopyramide assays.

### 2. Background Art

Disopyramide, or 4-diisopropylamino-2-phenyl-2-(2-pyridyl)-butyramide, is a drug used as an antiarrhythmic, to control abnormal rates or rhythms of the heart. It is frequently administered as a beta blocker. The drug can be toxic, and therapeutic concentrations may be close to toxic

levels. Since individuals vary in their response and drug concentration retention, monitoring the amount of the drug present in a patient's serum or plasma is desirable in order to maintain safe but effective levels of the drug.

One type of assay typically used for measuring ligands in a test sample, e.g. of a biological fluid, is the competitive binding immunoassay. (For the purposes of this disclosure, a "ligand" is a substance of biological interest to be quantitatively determined by an immunoassay technique.) The ligands compete with a labeled reagent, or "ligand analog," or "tracer," for a limited number of receptor binding sites on antibodies specific to the ligand and ligand analog. The concentration of ligand in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

In the past, however, patient serum/plasma disopyramide levels have been measured by enzyme immunoassays, or by liquid chromotography (HPLC) techniques. An improvement over such techniques would be a fluorescence polarization immunoassay for disopyramide which would enable more rapid assay of disopyramide and thus faster adjustment of therapeutic levels of disopyramide.

Fluorescence polarization techniques provide a quantitative means for measuring the amount of tracer-antibody conjugate produced in a competitive binding immunoassay. Fluorescence polarization techniques are based on the principle that certain compounds, when excited by plane polarized light, will emit fluorescence having a degree of polarization inversely related to their rate of rotation. Accordingly, when a fluorescent tracer-antibody conjugate labeled with such a fluorescent compound (fluorophore) is excited with plane polarized light, the emitted light remains highly polarized because the fluorophore is constrained from rotating between the time that light is absorbed and emitted. In contrast, when an unbound tracer compound is excited by plane polarized light, its rotation is much faster than the corresponding bound tracer-antibody conjugate and the molecules are more randomly oriented. As a result, the light emitted from the unbound tracer molecules is depolarized. Such fluorescence polarization techniques have been applied in U. S. Patent No. 4,420,568 to Wang et al., which is directed to the use of a triazinylaminofluorescein moiety as the fluorophore.

## SUMMARY OF THE INVENTION

The present invention provides an unexpected advance in the art beyond the aforementioned Wang patent, in that highly sensitive tracers, a method for making the tracers, and an assay using the tracers are provided specifically for the determination of disopyramide. In addition, an assay conducted in accordance with the present

invention is particularly accurate and sensitive, as explained in more detail below.

This invention is directed specifically to tracers for use in fluorescence polarization assays for disopyramide, to analytical methods for conducting such assays, an immunogen to raise antibody and to synthesis methods for making the tracers and immunogen.

A first aspect of the invention relatês to the discovery of unique tracers having novel structures. According to the first aspect of the invention, ten tracer compounds are provided for the fluorescence polarization assay of disopyramide. These are identified as Tracers Nos. 1-4 and 6-11. Comparative information for a similar compound, Compound No. 5, is also presented.

Tracer No. 1:

N-4-chloro-6-{p-[1-carbamyl-3-(diisopropylamino)-1-(2-pyridyl)propyl]anilino}triazin-2-yl-5-aminofluorescein.

Tracer No. 2:

N-4-chloro-6-{p-[1-carbamyl-3-(diisopropylamino)-1-(2-pyridyl)propyl]anilino}triazin-2-yl-6-aminofluorescein.

Tracer No. 3:

N-4-methoxy-6-{p-[1-carbamyl-3-(diisopropylamino)-1-(2-pyridyl)propyl]anilino}triazin-2-yl-5-aminofluorescein.

Tracer No. 4:

N-4-methoxy-6-{p-[1-carbamyl-3-(diisopropylamino)-1-(2-pyridyl)propyl]anilino}triazin-2-yl-6-aminofluorescein.

Compound No. 5:

N-{p-[1-carbamyl-3-(diisopropylamino)-1-(2-pyridyl)propyl]phenyl}-5-carbamylfluorescein.

Tracer No. 6:

N-{p-[1-carbamyl-3-(diisopropylamino)-1-(2-pyridyl)propyl]phenyl}-6-carbamylfluorescein.

Tracer No. 7:

N-4-chloro-6-{m-[1-carbamyl-3-(diisopropylamino)-1-(2-pyridyl)propyl]anilino}triazin-2-yl-5-aminofluorescein.

Tracer No. 8:

N-4-chloro-6-{m-[1-carbamyl-3-(diisopropylamino)-1-(2-pyridyl)propyl]anilino}triazin-2-yl-6-aminofluorescein.

Tracer No. 9:

N-4-methoxy-6-{m-[1-carbamyl-3-(diisopropylamino)-1-(2-pyridyl)propyl]anilino}triazin-2-yl-5-aminofluorescein.

Tracer No. 10:

N-4-methoxy-6-{m-[1-carbamyl-3-(diisopropylamino)-1-(2-pyridyl)propyl]anilino}triazin-2-yl-6-aminofluorescein.

Tracer No. 11:

N-{m-[1-carbamyl-3-(diisopropylamino)-1-(2-pyridyl)propyl]phenyl}-6-carbamylfluorescein.

A second aspect of the invention relates to the discovery of immunogens, which are the reaction product of p-aminodisopyramide with protein, to raise antibody.

The tracers described above are useful as reagents in the quantitative determination of the concentration of disopyramide in, e.g., blood serum or plasma. The immunogens are useful to raise antibody, for use with the tracer compounds.

A third aspect of the invention relates to synthesis methods, i. e. methods of making the tracer compounds and immunogens.

All of the 11 compounds listed above as Tracers Nos. 1-4 and 6-11, and Compound No. 5, are reaction products of p-aminodisopyramide, i. e. 4-diisopropylamino-2-(p-aminophenyl)-2-(2-pyridyl)butyramide, or m-aminodisopyramide, i.e. 4-diisopropylamino-2-(m-aminophenyl)-2-(2-pyridyl)butyramide, with another compound.

Tracer No. 1 is the reaction product of p-aminodisopyramide with 5-[(4,6-dichlorotriazin-2-yl)amino]-fluorescein, also known as "5-DTAF", "DTAF I" or "DTAF Form I".

Tracer No. 2 is the reaction product of p-aminodisopyramide with 6-[(4,6-dichlorotriazin-2-yl)amino]-fluorescein, also known as "6-DTAF", "DTAF II" or "DTAF Form II".

Tracer No. 3 is the reaction product of p-aminodisopyramide with 5-[(4-chloro-6-methoxytriazin-2-yl)-amino]fluorescein.

Tracer No. 4 is the reaction product of p-aminodisopyramide with 6-[(4-chloro-6-methoxytriazin-2-yl)-amino]fluorescein.

Compound No. 5 is the reaction product of p-aminodisopyramide with 5-carboxyfluorescein.

Tracer No. 6 is the reaction product of p-aminodisopyramide with 6-carboxyfluorescein.

Tracer No. 7 is the reaction product of m-aminodisopyramide with 5-[(4,6-dichlorotriazin-2-yl)amino]-fluorescein, also known as "5-DTAF", "DTAF I" or "DTAF Form I".

Tracer No. 8 is the reaction product of m-aminodisopyramide with 6-[(4,6-dichlorotriazin-2-yl)amino]-fluorescein, also known as "6-DTAF", "DTAF II" or "DTAF Form II".

Tracer No. 9 is the reaction product of m-aminodisopyramide with 5-[(4-chloro-6-methoxytriazin-2-yl)-amino]fluorescein.

Tracer No. 10 is the reaction product of m-aminodisopyramide with 6-[(4-chloro-6-methoxytriazin-2-yl)-amino]fluorescein.

Tracer No. 11 is the reaction product of m-aminodisopyramide with 6-carboxyfluorescein.

Structural formulas for Tracers Nos. 1-4 and 6-11, Compound No. 5 and the reactants employed to create them are shown in the drawings, as hereinafter described.

A fourth aspect of the invention relates to improved analytical methods of determining disopyramide by fluorescence immunoassay procedures, using the tracer compounds and immunogen of the invention. Tracers Nos. 1 and 2 are the preferred compounds for use in such analytical methods; and Tracer No. 1 is most preferred.

According to the fourth aspect of the invention, an improved fluorescence polarization assay is provided, comprising the steps of contacting a sample with a disopyramide antiserum and a fluorescent-labeled disopyramide derivative capable of producing a detectable fluorescence polarization response to the presence of disopyramide antiserum in a homogeneous solution, passing plane polarized light through the homogeneous solution, and measuring the fluorescence polarization response therefrom. Such an assay has been found to be unexpectedly advantageous for the reasons previously cited.

Further objects and advantages of the present invention will be understood from a reading of the following detailed description, taken together with the drawings and Examples.

BRIEF DESCRIPTION OF THE DRAWINGS

Figures 1-4, 8, 10 and 12 show the structural formulas and names of reactants used to form the products of the invention.

Figure 5 shows the alternate structural formulas and name of a precusor of reactants used to form the tracers of the invention.

Figures 6 and 7 show the structural formulas and names of intermediates used in forming the products of the invention.

Figures 9, 11 and 13-16 show the structural formulas and names of the tracers of the invention.

Figure 17 shows the structural formula and name of the analyte, or material being analyzed, in the analytical method aspect of the present invention.

Specifically:

Figures 1 and 2 illustrate the reactants p- and m-aminophenylacetonitrile.

Figure 3 illustrates the reactant 2-bromopyridine.

Figure 4 illustrates the reactant 2-chloroethyldiisopropylamine.

Figure 5 illustrates the alternate structural formulas of fluorescein, a precusor of reactants used to form the products of the invention.

Figure 6 illustrates the intermediates 4-diisopropylamino-2-(p- and m-aminophenyl)-2-(2-pyridyl)-butyronitrile, made as the reaction product of the compounds

shown in Figures 1 to 4. The broken line bonds indicate alternate locations (p- or m-) in Figs. 6 and 7.

Figure 7 illustrates the intermediates 4-diisopropylamino-2-(p- and m-aminophenyl)-2-(2-pyridyl)butyramide, made as the reaction product of the compounds shown in Figure 6 with sulfuric acid.

Figure 8 illustrates the reactants 5- and 6-[(4,6-dichlorotriazin-2-yl)amino]fluorescein. The broken line bond indicates alternate locations (carbon atoms 5 and 6, as numbered) of the fluorescein moiety at which the (4,6-dichlorotriazin-2-yl)amine moiety is joined.

Figure 9 illustrates two products of the invention, Tracers Nos. 1 and 2, N-4-chloro-6-{p-[1-carbamyl-3-(diisopropylamino)-1-(2-pyridyl)propyl]anilino}triazin-2-yl-5-aminofluorescein and N-4-chloro-6-{p-[1-carbamyl-3-(diisopropylamino)-1-(2-pyridyl)propyl]anilino}triazin-2-yl-6-aminofluorescein. The broken line bond indicates alternate locations (carbon atoms 5 and 6, as numbered) of the fluorescein moiety at which the p-aminodisopyramide moiety is joined.

Figure 10 illustrates the reactants 5- and 6-[(4-chloro-6-methoxytriazin-2-yl)aminofluorescein. The broken line bond indicates alternate locations (carbon atoms 5 and 6, as numbered) of the fluorescein moiety at which the p- or m-aminodisopyramide moiety is joined.

Figure 11 illustrates two products of the invention, Tracers Nos. 3 and 4, N-4-methoxy-6-{p-[1-carbamyl-3-

(diisopropylamino)-1-(2-pyridyl)propyl]anilino}triazin-2-yl-5-aminofluorescein and N-4-methoxy-6-{p-[1-carbamyl-3-(diisopropylamino)-1-(2-pyridyl)propyl]anilino}triazin-2-yl-6-aminofluorescein. The broken line bond indicates alternate locations (carbon atoms 5 and 6, as numbered) of the fluorescein moiety at which the p-aminodisopyramide moiety is joined.

Figure 12 illustrates the reactants 5- and 6-carboxyfluorescein. The broken line bond indicates alternate locations (carbon atoms 5 and 6, as numbered) of the fluorescein moiety at which the carboxylic acid moiety is joined.

Figure 13 illustrates Compound No. 5 and Tracer No. 6, N-{p-[1-carbamyl-3-(diisopropylamino)-1-(2-pyridyl)-propyl]phenyl}-5-carbamylfluorescein and N-{p-[1-carbamyl-3-(diisopropylamino)-1-(2-pyridyl)propyl]phenyl}-6-carbamyl-fluorescein. The broken line bond indicates alternate locations (carbon atoms 5 and 6, as numbered) of the fluorescein moiety at which the p-aminodisopyramide moiety is joined.

Figure 14 illustrates two products of the invention, Tracers Nos. 7 and 8, N-4-chloro-6-{m-[1-carbamyl-3-(diisopropylamino)-1-(2-pyridyl)propyl]anilino}triazin-2-yl-5-aminofluorescein and N-4-chloro-6-{m-[1-carbamyl-3-(diisopropylamino)-1-(2-pyridyl)propyl]anilino}triazin-2-yl-6-aminofluorescein. The broken line bond indicates alternate locations (carbon atoms 5 and 6, as numbered) of the fluorescein moiety at which the m-aminodisopyramide moiety is joined.

Figure 15 illustrates two products of the invention, Tracers Nos. 9 and 10, N-4-methoxy-m-{p-[1-carbamyl-3-(diisopropylamino)-1-(2-pyridyl)propyl]anilino}triazin-2-yl-5-aminofluorescein and N-4-methoxy-6-{p-[1-carbamyl-3-(diisopropylamino)-1-(2-pyridyl)propyl]anilino}triazin-2-yl-6-aminofluorescein. The broken line bond indicates alternate locations (carbon atoms 5 and 6, as numbered) of the fluorescein moiety at which the m-aminodisopyramide moiety is joined.

Figure 16 illustrates Tracer No. 11, N-{m-[1-carbamyl-3-(diisopropylamino)-1-(2-pyridyl)-propyl]phenyl}-6-carbamylfluorescein.

Figure 17 illustrates disopyramide, the analyte of the analytical method aspect of the present invention.

## DETAILED DESCRIPTION

The present invention involves the use of fluorescein derivatives as fluorescent tracer compounds. Fluorescein exists in 2 tautomeric forms, illustrated in Fig. 5, depending on the acid concentration (pH) of the environment of the fluorescein molecule. In the open (acid) form, there are a number of conjugated double bonds which make that form of fluorescein (and derivative compounds containing a fluorescein moiety) capable of absorbing blue light and emitting green fluorescence after an excited state lifetime

of about 4 nanoseconds. The open and closed (lactone) forms are easily converted to each other by adjustment of the pH level. Generally, the tracer compounds of the present invention exist in solution as biologically acceptable salts such as sodium, potassium, ammonium and the like, which allows the compounds to exist in the open, fluorescent form, when employed in the analytical methods of the present invention. The specific salt present will depend on the buffer employed to adjust the pH level. For example, in the presence of a sodium phosphate buffer, the compounds of the present invention will generally exist in the open form, as a sodium salt.

As used herein, the term "fluorescein," either as an individual compound or as a component of a larger compound, is meant to include both the open and closed forms, if they exist for a particular molecule, except in the context of fluorescence. An open form is necessary for the fluorescence to occur.

The numbering of carbon atoms of the fluorescein molecule varies, depending upon whether the open or closed form of the molecule is considered. Accordingly, the literature concerning fluorescein and its compounds is not uniform as to carbon atom numbering. The carbon atom of the phenyl group which is opposite the xanthene group is 4 for the open form and 5 for the closed form, while the adjacent carbon atom, opposite the carboxyl group, is conventionally

numbered 6 for the closed form of the compound, and 5 for the open form of the compound. For the purpose of this disclosure, the numbering of the closed form is adopted, because the raw materials used in the syntheses are most popularly numbered with that system. The carbon atom of fluorescein and its compounds which is opposite the carboxyl group is therefore numbered "6" for the purposes of the present disclosure.

A tracer which is not complexed (bound) to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization observed. Therefore, when a ligand competes with the tracer for antibody sites, the observed polarization of fluorescence of the tracer-antibody complex becomes a value somewhere between that of the tracer and tracer-antibody complex. If a sample contains a high concentration of the ligand, the observed polarization value is closer to that of the free ligand, i.e., low. If the test sample contains a low concentration of the ligand, the polarization value is

closer to that of the bound ligand, i.e., high. By sequentially exciting the reaction mixture of an immunoassay with vertically and then horizontally polarized light and analyzing only the vertical component of the emitted light, the polarization of fluorescence in the reaction mixture may be accurately determined. The precise relationship between polarization and concentration of the ligand to be determined is established by measuring the polarization values of calibrators with known concentrations. The concentration of the ligand can be interpolated or extrapolated from a standard curve prepared in this manner.

The particular tracers formed in accordance with this invention have been found to produce surprisingly good results in fluorescence polarization assays.

The preparation of the compounds of the present invention will now be illustrated with examples.

EXAMPLE 1

Preparation of

p-Aminodisopyramide

To a mixture of 5.2 grams of p-aminophenylacetonitrile (see Fig. 1) and 6.5 grams of 2-bromopyridine (see Fig. 3) in 43.5 ml of dry toluene at 80 degrees C was added 3.3 grams of sodium amide, in portions over 1 hour, the temperature remaining between 80 and 85 degrees C during this time. The reaction mixture was then heated with stirring at

105 degrees C for one hour. To this mixture was added 8.2 grams of 2-chloroethyldiisopropylamine (see Fig. 4) in 13 ml of dry toluene. The resultant mixture was heated at 110+2 degrees C for 3.5 hours. The dark mixture was cooled to room temperature and quenched with 20 ml of water. The layers were separated. The toluene fraction was dried with sodium sulfate, filtered and concentrated in vacuo to a viscous oil. Chromatography on 150 grams of silica gel and elution with a solvent system consisting of 94.5% chloroform, 5% ethanol and 0.5% concentrated ammonium hydroxide yielded 2.72 grams of pure product, identified by nuclear magnetic resonance as 4-diisopropylamino-2-(p-aminophenyl)-2-(2-pyridyl)butyronitrile (see Fig. 6).

In the above synthesis, potassium amide may be substituted for the sodium amide.

A mixture of 0.72 grams of 4-diisopropylamino-2-(p-aminophenyl)-2-(2-pyridyl)butyronitrile, made as indicated above, and 14 ml of concentrated sulfuric acid was heated at 100+2 degrees C for 4 hours. The solution was cooled to room temperature and the poured onto ice. The resultant mixture was made basic (pH about 11) with NaOH. The mixture was extracted with 4 portions, 25 ml each, of chloroform. The extracts were dried with magnesium sulfate, filtered, and concentrated in vacuo to yield a gummy solid. The solid was chromatographed on 25 grams of silica gel, and eluted with a solvent system consisting of 83.5% chloroform, 15%

methanol and 1.5% concentrated ammonium hydroxide to yield 0.356 grams of pure product. Repetition of the same procedure yielded 1.07 grams of pure product, identified by nuclear magnetic resonance as 4-diisopropylamino-2-(p-aminophenyl)-2-(2-pyridyl)butyramide (see Fig. 7). The compound is also known as p-aminodisopyramide,or disopyramide hapten.

EXAMPLE 2

Preparation of Tracer No. 1

(N-4-chloro-6-{p-[1-carbamyl-3-(diisopropylamino)-1-(2-pyridyl)propyl]anilino}triazin-2-yl-5-aminofluorescein)

A solution of 5 milligrams of p-aminodisopyramide, prepared as in Example 1, 8 milligrams of 5-[(4,6-dichlorotriazin-2-yl)amino]fluorescein ("DTAF I", a commercially available material, see Fig. 8) and 0.5 ml of methanol was stirred at room temperature for 18 hours. The crude reaction mixture was placed on a thin layer chromatography 0.5 mm silica gel plate and eluted with a mixture of 50 parts chloroform, 50 parts methanol and 0.5 parts acetic acid. The product was eluted from the silica with methanol, repurified on a 0.5 mm silica gel plate and again eluted with a mixture of 50 parts chloroform, 50 parts methanol and 0.5 parts acetic acid. The major phosphorescent band was taken and eluted from the silica with methanol. The product was Tracer No. 1, N-4-chloro-6-{p-[1-carbamyl-3-(diisopropylamino)-1-(2-pyridyl)propyl]anilino}triazin-2-yl-5-aminofluorescein (see Fig. 9).

## EXAMPLE 3

### Preparation of 6-[(4,6-dichlorotriazin-2-yl)amino]fluorescein ("DTAF II")

6-[(4,6-dichlorotriazin-2-yl)amino]fluorescein ("DTAF II", see Fig. 7) can be synthesized from 6-aminofluorescein and 2,4,6-trichlorotriazine, using the procedures described in Examples 5 or 7, below.

## EXAMPLE 4

### Preparation of Tracer No. 2 (N-4-chloro-6-{p-[1-carbamyl-3-diisopropylamino)-1-(2-pyridyl)propyl]anilino}triazin-2-yl-6-aminofluorescein).

Example 2 was repeated, substituting 6-[(4,6-dichlorotriazin-2-yl)amino]fluorescein ("DTAF II") for the DTAF I used in Example 2. The product was Tracer No. 2, N-4-chloro-6-{p-[1-carbamyl-3-diisopropylamino)-1-(2-pyridyl)propyl]anilino}triazin-2-yl-6-aminofluorescein (see Fig. 9).

## EXAMPLE 5

### Preparation of 5-[(4-chloro-6-methoxytriazin-2-yl)amino]fluorescein

5-[(4-chloro-6-methoxytriazin-2-yl)amino]-fluorescein (see Fig. 10) was synthesized as follows:

One gram of 5-aminofluorescein was stirred in 50 ml of methanol and the mixture was separated into undissolved material and solution. The mother liquid was decanted into a 250 ml round-bottom flask and cooled to 4 degrees C with an ice bath. Then a solution of 2,4-dichloro-6-methoxytriazine in 5 ml of chloroform was added dropwise at 4 degrees C and the mixture stirred for an additional 60 minutes. 1 ml of concentrated hydrochloric acid was added to the mixture. A yellow solid formed upon addition of the hydrochloric acid. The stirring was continued for another 60 minutes and the yellow precipitate was filtered and dried. The mother liquid was evaporated to dryness to afford a second crop, yielding 5-[(4-chloro-6-methoxytriazin-2-yl)amino]fluorescein (see Fig. 10) in 85% yield.

EXAMPLE 6

Preparation of Tracer No. 3 (N-4-methoxy-6-{p-[1-carbamyl-3-diisopropylamino)-1-(2-pyridyl)propyl]anilino}triazin-2-yl-5-aminofluorescein).

Example 2 was again repeated, substituting the product of Example 5 for the DTAF I used in Example 2, and stirring for 24 hours instead of 18. The product was Tracer No. 3, N-4-methoxy-6-{p-[1-carbamyl-3-diisopropylamino)-1-(2-pyridyl)propyl]anilino}triazin-2-yl-5-aminofluorescein (see Fig. 11).

EXAMPLE 7

Preparation of

6-[(4-chloro-6-methoxytriazin-2-yl)amino]fluorescein

6-[(4-chloro-6-methoxytriazin-2-yl)amino]-fluorescein (see Fig. 10) was synthesized as follows:

0.5 grams 6-aminofluorescein in 5 ml of methanol at 0 degrees C was filtered through a cotton wad into 0.35 grams of 2,4-dichloro-6-methoxytriazine in 2.5 ml of chloroform at 0 degrees C. The addition took about 1 hour. After 4 hours at 0 degrees C a solid had formed. 0.5 ml of concentrated hydrochloric acid was added. After stiring a few minutes the solid was filtered, washed with dichloromethane and air dried. 0.55 grams of yellow product, 6-[(4-chloro-6-methoxytriazin-2-yl)amino]fluorescein (see Fig. 10), was obtained.

EXAMPLE 8

Preparation of Tracer No. 4

(N-4-methoxy-6-{p-[1-carbamyl-3-(diisopropylamino)-1-(2-pyridyl)propyl]anilino}triazin-2-yl-6-aminofluorescein)

Example 2 was again repeated, substituting the product of Example 7 for the DTAF I used in Example 2 and again stirring for 24 hours instead of 18. The product was Tracer No. 4, N-4-methoxy-6-{p-[1-carbamyl-3-(diisopropyl-amino)-1-(2-pyridyl)propyl]anilino}triazin-2-yl-6-amino-fluorescein (see Fig. 11).

EXAMPLE 9

Preparation of Compound No. 5

(N-{p-[1-carbamyl-3-(diisopropylamino)-1-(2-pyridyl)propyl]phenyl}-5-carbamylfluorescein)

A solution of 11.2 mgm of 5-carboxyfluorescein, 3.6 mgm of N-hydroxysuccinimide and 6.4 mgm of 1,3-dicyclohexylcarbodiimide in 0.5 ml of dry pyridine was stirred at room temperature for 2 hours. A solution of 10.0 milligrams of p-aminodisopyramide, prepared as in Example 1, in 0.25 ml of dry pyridine was added. The solution was left to stir at room temperature for 18 hours. The sclution was placed on an 0.5 mm x 20 cm x 20 cm silica gel plate and eluted with chloroform/methanol/acetic acid in a ratio of 60/40/0.4. The product was Compound No. 5, N-{p-[1-carbamyl-3-(diisopropylamino)1-(2-pyridyl)propyl]-phenyl}-5-carbamylfluorescein (see Fig. 13).

EXAMPLE 10

Preparation of Tracer No. 6

(N-{p-[1-carbamyl-3-(diisopropylamino)-1-(2-pyridyl)propyl]phenyl}-6-carbamylfluorescein)

Example 9 was repeated, substituting 6-carboxyfluorescein for the 5-carboxyfluorescein. The product was Tracer No. 6, N-{p-[1-carbamyl-3-(diisopropylamino)-1-(2-pyridyl)propyl]phenyl}-6-carbamylfluorescein (see Fig. 13).

EXAMPLE 11

Preparation of

m-Aminodisopyramide

Example 1 is repeated, substituting m-aminophenylacetonitrile for the p-aminophenylacetonitrile. The product is 4-diisopropylamino-2-(m-aminophenyl)-2-(2-pyridyl)-butyramide (See Figs. 2 and 7). The compound is also known as m-aminodisopyramide.

EXAMPLE 12

Preparation of Tracer No. 7

(N-4-chloro-6-{m-[1-carbamyl-3-(diisopropylamino)-1-(2-pyridyl)propyl]anilino}triazin-2-yl-5-aminofluorescein)

Example 2 is repeated, substituting m-aminodisopyramide for the p-aminodisopyramide. The product is Tracer No. 7, (N-4-chloro-6-{m-[1-carbamyl-3-(diisopropylamino)-1-(2-pyridyl)propyl]anilino}triazin-2-yl-5-aminofluorescein (See Fig. 14).

EXAMPLES 13-16

Examples 4, 6, 8 and 10 are repeated, substituting m-aminodisopyramide for the p-aminodisopyramide.

The product of Example 13 is Tracer No. 8, N-4-chloro-6-{m-[1-carbamyl-3-(diisopropylamino)-1-(2-pyridyl)propyl]anilino}triazin-2-yl-6-aminofluorescein (See Fig. 14).

The product of Example 14 is Tracer No. 9, N-4-methoxy-6-{m-[1-carbamyl-3-(diisopropylamino)-1-(2-pyridyl)propyl]anilino}triazin-2-yl-5-aminofluorescein (See Fig. 15).

The product of Example 15 is Tracer No. 10, N-4-methoxy-6-{m-[1-carbamyl-3-(diisopropylamino)-1-(2-pyridyl)propyl]anilino}triazin-2-yl-6-aminofluorescein (See Fig. 15).

The product of Example 16 is Tracer No. 11, N-{m-[1-carbamyl-3-(diisopropylamino)-1-(2-pyridyl)-propyl]phenyl}-6-carbamylfluorescein (See Fig. 16).

## EXAMPLE 17

### Preparation of Immunogen

Four solutions are prepared as follows:

0.1 M sodium borate buffer: 381 milligrams of sodium borate decahydrate is dissolved in 8 ml of water, the pH adjusted to 9.0 with 1 M hydrochloric acid, and sufficient water to form 10 ml of solution is added.

1 M sodium nitrite: 69 milligrams of sodium nitrite is dissolved in 1 ml water.

1 M urea: 60 milligrams of urea is dissolved in 1 ml of water.

0.05 M sodium bicarbonate: 8.4 grams of sodium bicarbonate is dissolved in 2 liters of water.

40 milligrams of p-aminodisopyramide, prepared as in Example 1, is dissolved in 2 ml of dimethyl formamide. 0.6 ml of water is added. The solution is cooled to 2-4 degrees C in an ice bath and 0.4 ml of 1 N hydrochloric acid is added. 0.12 ml of a fresh 1 M solution of sodium nitrite in water is added, and the mixture is stirred for 30 minutes, maintaining the temperature at 2-4 degrees C. 0.1 ml of 1 M urea is added to decompose nitrous acid. The presence of nitrous acid is checked by placing a drop of the solution on a piece of starch-iodide paper. The paper should not turn to a blue-black color.

The above solution is added dropwise to a 2-4 degree C solution of bovine serum albumin in 5 ml of 0.1 M borate buffer having a pH of 9. The pH is monitored for 15 minutes and the pH adjusted to 9.0 with 1 N sodium hydroxide or 1 N hydrochloric acid, if necessary.

The solution is stirred overnight at 2-4 degrees C, dialyzed twice with 2 liters of 0.05 M sodium bicarbonate, and twice more with 2 liters of water. The product is the reaction product of p-aminodisopyramide with the bovine serum albumin protein, and is stored frozen.

The above procedure was carried out as stated, then repeated with twice the amount of p-aminodisopyramide.

Based on absorbance data, the product was calculated to be the reaction product of 12 parts p-aminodisopyramide with one of bovine serum albumin protein.

Other protein materials can be substituted for the bovine serum albumin protein with similar results. Likewise, m-aminodisopyramide can be substituted for the p-aminodisopyramide.

The reaction conditions described above can be varied according to conditions or individual preference. The reactions can be caried out at temperatures from about 0 degrees C to about 100 degrees C, most preferably between 0 and 80. Reaction times vary with reaction temperatures, between about 0.5 hour and 6 days.

In accordance with the analytical methods of the present invention, i. e. the methods of determining disopyramide by a fluorescence immunoassay procedure using the tracer compounds and immunogens of the invention, a sample containing or suspected of containing disopyramide is intermixed with a biologically acceptable salt of one of Tracers Nos. 1 to 4 or 6 to 11 (preferable Tracer No. 1), and an antibody specific to disopyramide and the tracer. The antibody is produced using the immunogen as described in Example 17. The disopyramide and tracer compete for limited antibody sites, resulting in the formation of complexes. By maintaining constant the concentration of tracer and antibody, the ratio of disopyramide-antibody complex to tracer-antibody complex that is formed is directly proportional to the amount of disopyramide in the sample. Therefore, upon exciting the mixture with linearly polarized

light and measuring the polarization of the fluorescence emitted by a tracer and a tracer-antibody complex, one is able to quantitatively determine the amount of disopyramide in the sample.

The pH at which the method of the present invention is practiced must be sufficient to allow the fluorescein moiety of the tracers to exist in their open form. The pH may range from about 3 to 12, more usually in the range of from about 5 to 10, most preferably from about 6 to 9. Various buffers may be used to achieve and maintain the pH during the assay procedure. Representative buffers include borate, phosphate, carbonate, tris, barbital and the like. The particular buffer employed is not critical to the present invention, but the tris and phosphate buffers are preferred. The cation portion of the buffer will generally determine the cation portion of the tracer salt in solution.

The reagents for the fluorescence polarization assay of the present invention comprise antibody specific for disopyramide and one of the tracers herein described. Additionally, a disopyramide pretreatment solution, a dilution buffer, disopyramide calibrators and disopyramide controls are desirably prepared.

The tracer formulation presently preferred is 80.0 nanomolar tracer in: 0.1 molar tris buffer at pH 7.5; 15% (weight/volume) cholic acid sodium salt; and 0.1% sodium azide. The antiserum formulation comprises rabbit serum

diluted with 0.9% (weight/volume) sodium chloride and 0.1% (weight/volume) sodium azide. The dilution buffer comprises: 0.1 molar sodium phosphate at pH 7.5; 0.1% (weight/volume) sodium azide; and 0.01% (weight/volume) bovine gamma globulin. The pretreatment formulation comprises: 0.1 molar tris buffer at pH 7.5; 0.1% (weight/volume) sodium azide; and 15% (weight/volume) cholic acid sodium salt. Disopyramide calibrators comprising disopyramide and normal human serum at concentrations of 0.0, 0.5, 1.0, 2.0, 4.0 and 8.0 micrograms per milliliter, with 0.1% sodium azide preservative are useful. Disopyramide controls comprising disopyramide and normal human serum are provided at concentrations of 1.5 3.0 and 6.0 micrograms per milliliter, with 0.1% sodium azide preservative are also useful.

The preferred method of the improved assay of the present invention will now be discussed in detail. The assay is a "homogeneous assay," which means that the end polarization readings are taken from a solution in which bound tracer is not separated from unbound tracer. This is a distinct advantage over radioimmunoassay procedures, for example, where the bound radioactive tracer must be separated from the unbound radioactive tracer before a reading can be taken.

According to the preferred assay procedure of the present invention, disopyramide calibrators, controls and unknown samples must be prepared by the same procedure. In

the preferred procedure, which is designed to be used in conjunction with the Abbott TDx® Polarization Analyzer, available from Abbott Laboratories, Abbott Park, Illinois, 50 microliters of the serum or plasma sample are pipetted into the sample well of the TDx® sample cartridge or equivalent. Once the reagent pack and the carousel are placed in the instrument, the assay is automatically performed by pressing the "run" button.

If a manual assay is being performed, then the sample is mixed with dilution buffer. The antibody and the pretreatment solution are placed into the test tube containing the sample, and a background reading is taken. Then tracer and dilution buffer are added to the sample, and, after incubation, a fluorescence polarization reading is taken.

The fluorescence polarization value of each calibrator, control or sample is determined and is printed on the output tape of an instrument such as the Abbott TDx® Polarization Analyzer. A standard curve is generated in the instrument by plotting the polarization, P, of each calibrator versus its concentration using a nonlinear regression analysis. The concentration of each control and samples is read off the stored calibration curve and printed on the output tape.

With respect to the foregoing preferred procedure, it should be noted that the disopyramide tracer, antibody,

pretreatment solution, calibrators and controls should be used directly after removal from storage at between about 2 and about 8°C, while the dilution buffer should be stored at room temperature. A standard curve and controls should be run every two weeks, with each calibrator and control run in duplicate. Controls should be run with each batch, and all samples can be run in duplicate.

Tracers Nos. 1-4 and 6 and Compound No. 5 have been tested for effectiveness in fluorescence polarization immunoassay determination of disopyramide. The results are set forth in Table I, below.

The results can be quantified in terms of net millipolarization units, span (in millipolarization units) and intensity. The measurement of millipolarization units indicates the maximum polarization when a maximum amount of the tracer is bound to the antibody in the absence of any disopyramide. The higher the net millipolarization units, the better the binding of the tracer to the antibody. The span is an indication of the difference between the net millipolarization and the minimum amount of tracer bound to the antibody. A larger span provides for a better numerical analysis of data. The intensity is a measure of the strength of the signal above background. Thus, a higher intensity will give a more accurate measurement. The intensity is determined at about 1 nanomolar (i.e., approximately 0.8-1.2 nanomolar) concentration of tracer as the sum

of the vertically polarized intensity plus twice the horizontally polarized intensity.

Table I shows that the results of the tracers of the present invention, in terms of net millipolarization units, span and intensity, are far superior to the results obtained as compared to Compound No. 5. The surprising aspect is that one would not expect that the tracers of the present invention would be well recognized by the disopyramide specific antiserum, because of the substantial differences in structure between the relatively small disopyramide and tracer molecules.

TABLE I

| TRACER OR COMPOUND | MAXIMUM BINDING (mp) | SPAN (mp) | INTENSITY |
|---|---|---|---|
| Tracer #1 | 180 | 140 | 4500-6500 |
| Tracer #2 | 170 | 115 | 5400-7400 |
| Tracer #3 | 190 | 146 | 4500-5900 |
| Tracer #4 | 200 | 117 | 4700-5200 |
| Compound #5 | 3 | (not determined) | 13,000-14,000 |
| Tracer #6 | 170-205 | (not determined) | 6000-7500 |

In each case, chromatographic separation of the synthesized compounds resulted in a plurality of chromatographic bands, which were tested separately. The data

presented in Table I is for the best band observed for the particular tracer compound listed, or a combination of values observed for several bands.

The analytical method of the present invention allows more rapid assay work with fluorescence polarization than the enzyme immunoassays of the prior art. The present analytical method also has lower crossreactivity with the major metabolite of disopyramide than such prior art assays.

It should be understood that the foregoing detailed description and the Examples are intended to be illustrative, but not limiting, with respect to the scope of the present invention. Various modifications will become apparent to one skilled in the art, and thus it is intended that the scope of the invention be defined by the claims and equivalents thereto.

CLAIMS

1. A compound selected from the group consisting of N-4-chloro-6-(p-[1-carbamyl-3-(diisopropylamino)-1-(2-pyridyl)propyl] anilino)triazin-2-yl-5-aminofluorescein, N-4-chloro-6-(p-[1-carbamyl 3-(diisopropylamino)-1-(2-pyridyl)propyl]anilino)triazin-2-yl-6-aminofluorescein, N-4-methoxy-6-(p-[1-carbamyl-3-(diisopropylamino) -1-(2-pyridyl)propyl]anilino)triazin-2-yl-5-aminofluorescein, N-4-methoxy-6-(p-[1-carbamyl-3-(diisopropylamino)-1-(2-pyridyl)propyl] anilino)triazin-2-yl-6-aminofluorescein, N-(p-[1-carbamyl-3-diisopropylamino)-1-(2-pyridyl)propyl]phenyl)-6-carbamylfluorescein, N-4-chloro-6-(m-[1-carbamyl-3-(diisopropylamino)-1-(2-pyridyl)propyl] anilino)triazin-2-yl-5-aminofluorescein, N-4-chloro-6-(m-[1-carbamyl -3-(diisopropylamino)-1-(2-pyridyl)propyl]anilino)triazin-2-yl-6-aminofluorescein, N-4-methoxy-6-(m-[1-carbamyl-3-(diisopropylamino) -1-(2-pyridyl)propyl]anilino)triazin-2-yl-5-aminofluorescein, N-4-methoxy-6-(m-[1-carbamyl-3-(diisopropylamino)-1-(2-pyridyl)propyl] anilino)triazin-2-yl-6-aminofluorescein, N-(m-[1-carbamyl-3-(diisopropylamino)-1-(2-pyridyl)propyl]phenyl)-6-carbamylfluorescein, having formulas as set forth in Figures 9 through 16.

2. The product of the reaction of p-aminodisopyramide or m-aminodisopyramide with a derivative of fluorescein.

3. A product according to Claim 11, wherein the derivative of fluorescein is selected from the group consisting of 5-[(4,6-dichlorotriazin-2-yl)-amino-fluorescein, 6-[(4,6-dichlorotriazin-2-yl)-amino]-fluorescein, 5-[(4-chloro-6-methoxytriazin-2-yl)-amino]-fluorescein, 6-[(4-chloro-6-methoxytriazin-2-yl)-amino]fluorescein and 6-carboxyfluorescein.

4. The product of the reaction of p-aminodisopyramide or m-aminodisopyamide with protein.

5. A method of making a compound according to Claim 11, by reacting p-aminodisopyramide or m-aminodisopyramide with a compound selected from the group consisting of 5-[(4,6-dichlorotriazin-2-yl)amino]fluorescein, 6-[(4,6-dichlorotriazin-2-yl)amino]fluorescein, 5-[(4-chloro-6-methoxytriazin-2-yl)amino]fluorescein, 6-[(4-chloro-6-methoxytriazin-2-yl)amino]fluorescein and 6-carboxyfluorescein.

6. A process for measuring the concentration of disopyramide in a sample which comprises:

(a) contacting the sample with a p- or m-aminodisopyramide antiserum;

(b) contacting the resulting solution with a fluorescein-p- or m-aminodisopyramide derivative capable of producing a measureable fluorescence polarization response to the presence of p- or m-aminodisopyramide antiserum and of forming a homogeneous test solution;

(c) passing plane polarized light through the homogeneous test solution to obtain a fluorescence polarization response;

(d) measuring the fluorescence polarization response of the homogeneous test solution; and

(e) comparing the measured fluorescence polarization response to that obtained with standard samples, thereby to determine the concentration of disopyramide in the sample.

7. A process according to Claim 15 wherein the fluorescein-p- or m-aminodisopyramide derivative is selected from the group consisting of N-4-chloro-6-(p-[1-carbamyl-3-(diisopropylamino)-1-(2-pyridyl)propyl] anilino)triazin-2-yl-5-aminofluorescein; N-4-chloro-6-(p-[1-carbamyl -3-(diisopropylamino)-1-(2-pyridyl)propyl]anilino)triazin-2-yl-6-aminofluorescein; N-4-methoxy-6-(p-[1-carbamyl-3-(diisopropylamino) -1-(2-pyridyl)propyl]anilino)triazin-2-yl-5-aminofluorescein; N-4-methoxy-6-(p-[1-carbamyl-3-(diisopropylamino)-1-(2-pyridyl)propyl] anilino)triazin-2-yl-6-aminofluorescein; and N-(p-[1-carbamyl-3-(diisopropylamino)-1-(2-pyridyl)propyl]phenyl)-6-carbamyl-fluorescein.

R. L. WALTERS ET AL.



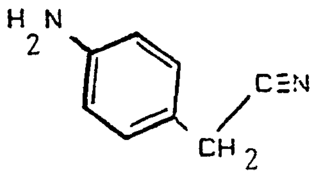

p-AMINOPHENYLACETONITRILE.

(REACTANT)

FIGURE 1.

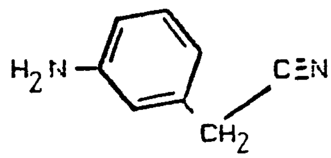

m-AMINOPHENYLACETONITRILE.

(REACTANT)

FIGURE 2.

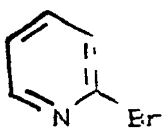

2-BROMOPYRIDINE.

(REACTANT)

FIGURE 3.

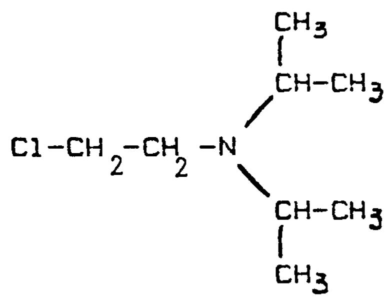

2-CHLOROETHYLDIISOPROPYLAMINE.

(REACTANT)

FIGURE 4.

(5)

(6)

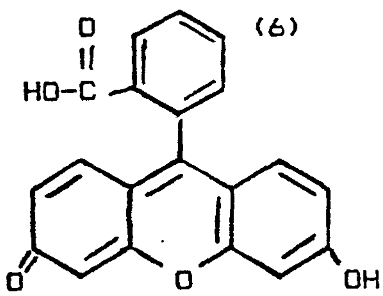

OPEN (ACID) FORM

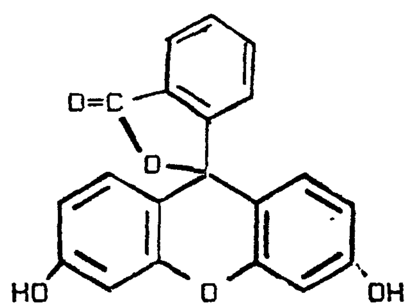

CLOSED (LACTONE) FORM

FLUORESCEIN.

(PRECURSOR)

FIGURE 5.

R. L. WALTERS ET AL.

SHEET 2 OF 7

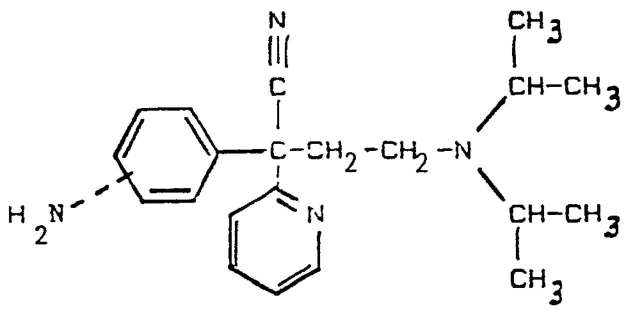

4-DIISOPROPYLAMINO-2-(p- or m-AMINOPHENYL)-

2-(2-PYRIDYL)BUTYRONITRILE.

(INTERMEDIATE)

FIGURE 6.

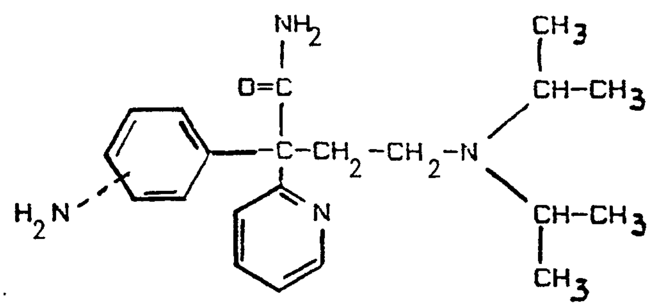

4-DIISOPROPYLAMINO-2-(p- or m-AMINOPHENYL)-

2-(2-PYRIDYL)BUTYRAMIDE.

("p- (or m- ) AMINODISOPYRAMIDE")

(INTERMEDIATE)

FIGURE 7.

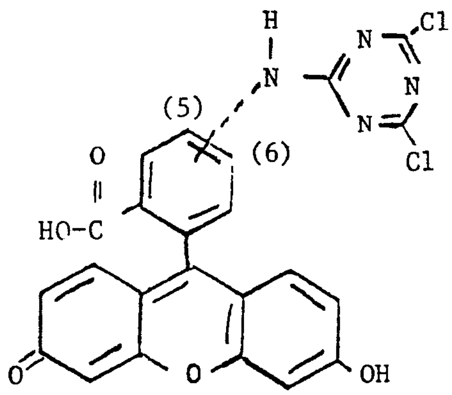

5- or 6-[(4,6-DICHLOROTRIAZIN-2-YL)AMINO]FLUORESCEIN.

("DTAF")

(REACTANT)

FIGURE 8.

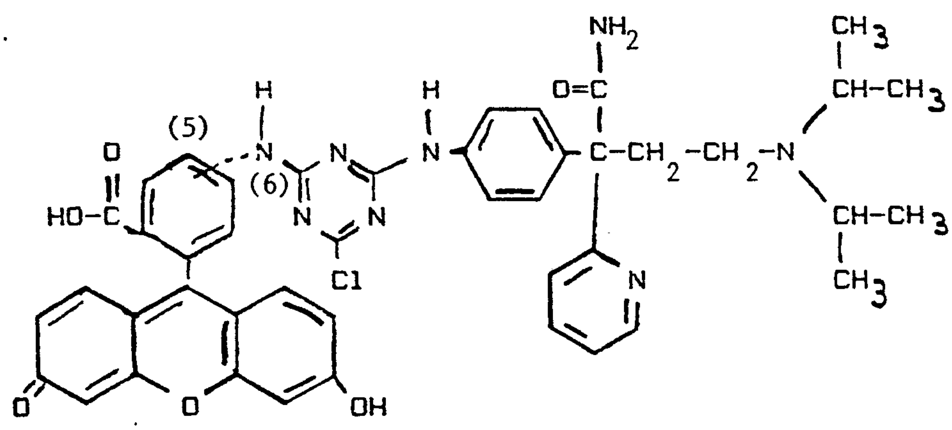

N-4-CHLORO-6-{p-[1-CARBAMYL-3-(DIISOPROPYLAMINO)-1-(2-PYRIDYL)PROPYL]ANILINO}TRIAZIN-2-YL-x-AMINOFLUORESCEIN.

(x=5, TRACER NO. 1; x=6, TRACER NO. 2)

FIGURE 9.

C184120

4/7

R. L. WALTERS ET AL.

SHEET 4 OF 7

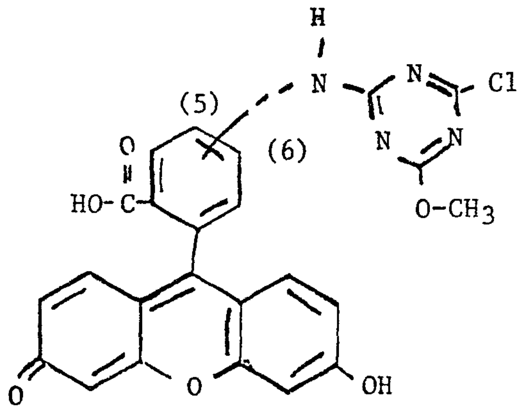

5- or 6-[(4-CHLORO-6-METHOXYTRIAZIN-2-YL)AMINO]FLUORESCEIN.

(REACTANT)

FIGURE 10.

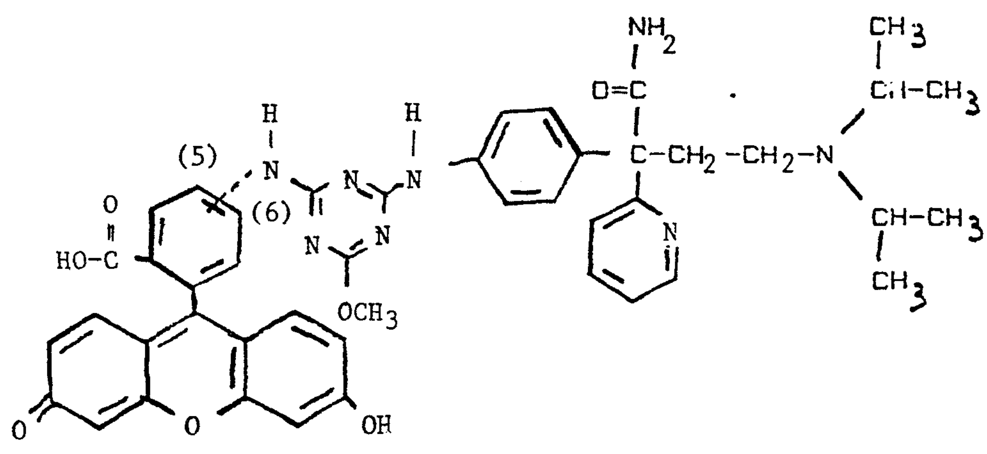

N-4-METHOXY-6-{p-[1-CARBAMYL-3-(DIISOPROPYLAMINO)-1-(2-PYRIDYL)PROPYL]ANILINO}TRIAZIN-2-YL-x-AMINOFLUORESCEIN.

(x=5, TRACER NO. 3; x=6, TRACER NO. 4)

FIGURE 11.

R. L. WALTERS ET AL.

SHEET 5 OF 7

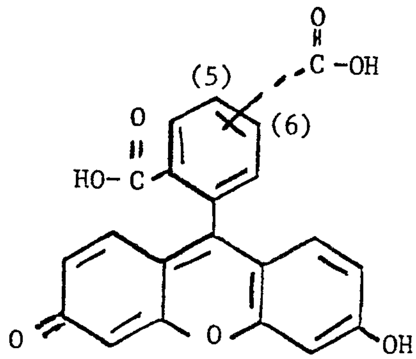

5- or 6-CARBOXYFLUORESCEIN.

(REACTANT)

FIGURE 12.

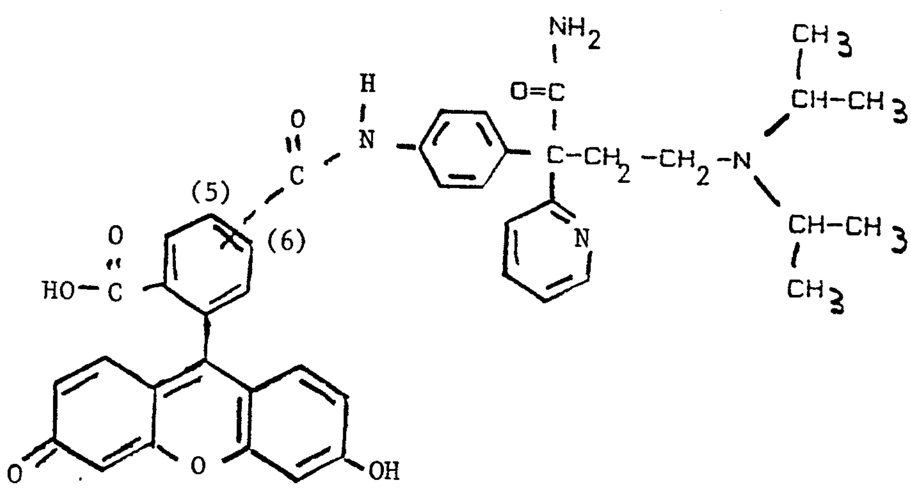

N-{p-[1-CARBAMYL-3-(DIISOPROPYLAMINO)-1-(2-PYRIDYL)PROPYL]PHENYL}-x-CARBAMYLFLUORESCEIN.

(x=5, COMPOUND NO. 5; x=6, TRACER NO. 6)

FIGURE 13.

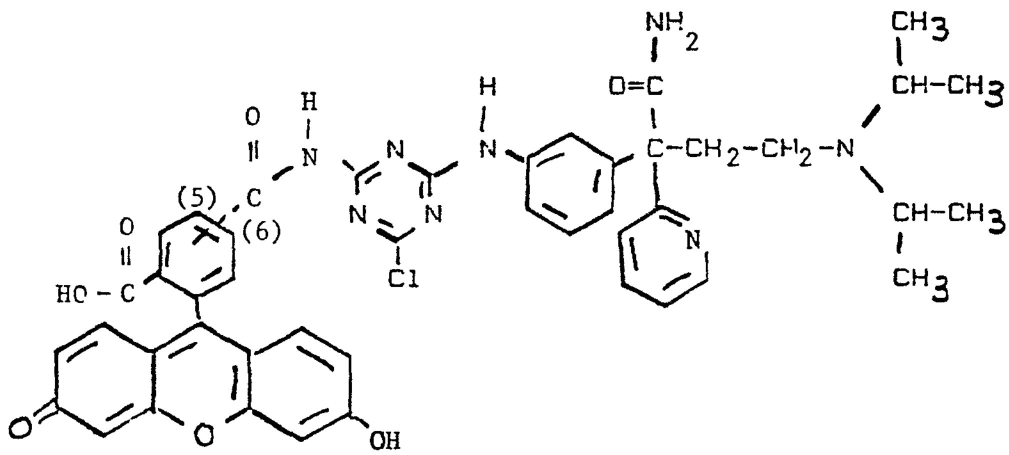

N-4-CHLORO-6-{m-[1-CARBAMYL-3-(DIISOPROPYLAMINO)-1-(2-PYRIDYL)PROPYL]ANILINO}TRIAZIN-2-YL-x-AMINOFLUORESCEIN.

(x=5, TRACER NO. 7; x=6, TRACER NO. 8)

FIGURE 14.

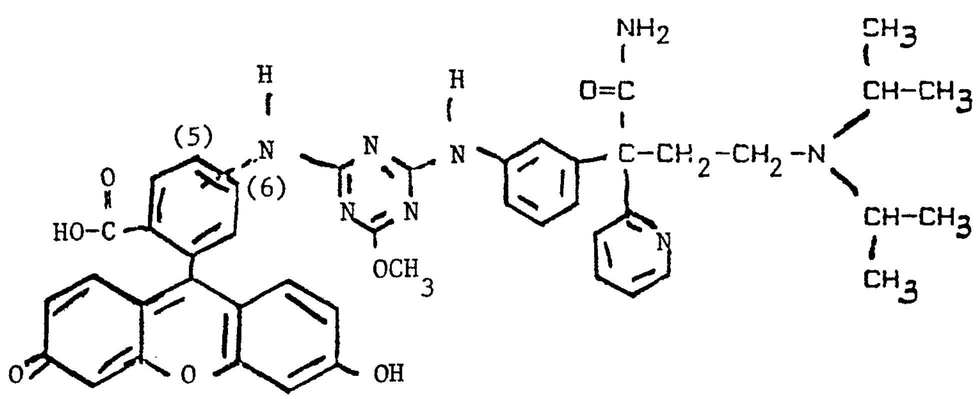

N-4-METHOXY-6-{m-[1-CARBAMYL-3-(DIISOPROPYLAMINO)-1-(2-PYRIDYL)PROPYL]ANILINO}TRIAZIN-2-YL-x-AMINOFLUORESCEIN.

(x=5, TRACER NO. 9; x=6, TRACER NO. 11)

FIGURE 15.

R. L. WALTERS ET AL.

SHEET 7 OF 7

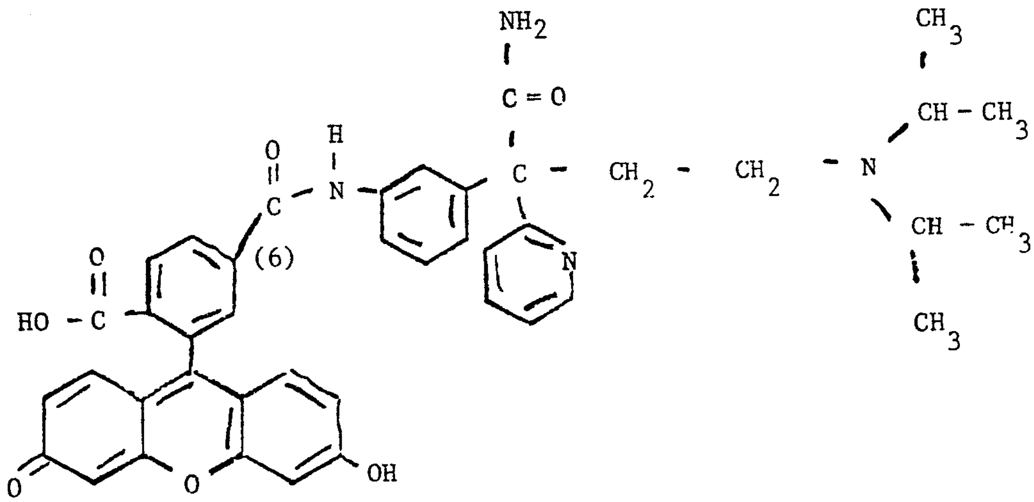

N-{m-[1-CARBAMYL-3-(DIISOPROPYLAMINO)-1-(2-PYRIDYL)PROPYL]PHENYL}-6-CARBAMYLFLUORESCEIN.

(TRACER NO. 11)

FIGURE 16.

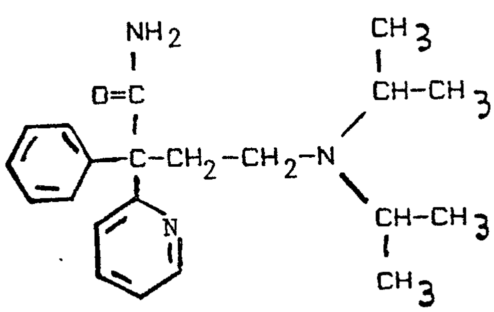

DISOPYRAMIDE.

(ANALYTE)

FIGURE 17.